(19) Europäisches Patentamt / European Patent Office / Office européen des brevets

(11) **EP 3 892 189 B1**

(12) **EUROPEAN PATENT SPECIFICATION**

(45) Date of publication and mention
of the grant of the patent:
**07.06.2023 Bulletin 2023/23**

(21) Application number: **20315124.6**

(22) Date of filing: **06.04.2020**

(51) International Patent Classification (IPC):
**A61B 5/0205** $^{(2006.01)}$    **A61B 5/024** $^{(2006.01)}$
**A61B 5/0245** $^{(2006.01)}$    **A61B 5/1455** $^{(2006.01)}$
**A61B 5/00** $^{(2006.01)}$

(52) Cooperative Patent Classification (CPC):
**A61B 5/0205; A61B 5/02438; A61B 5/0245;**
**A61B 5/14552; A61B 5/681;** A61B 2562/063;
A61B 2562/185

(54) **WRISTWATCH FOR MEASURING SPO2**

ARMBANDUHR ZUR SPO2-MESSUNG

MONTRE POUR MESURER LE SPO2

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**13.10.2021 Bulletin 2021/41**

(73) Proprietor: **Withings**
**92130 Issy-les-Moulineaux (FR)**

(72) Inventors:
• **Kirszenblat, Romain**
**92130 Issy LES Moulineaux (FR)**
• **Bendjoudi, Aniss**
**92130 Issy LES Moulineaux (FR)**
• **Rafik, Walid**
**92130 Issy LES Moulineaux (FR)**
• **Oueslati, Ianis**
**92130 Issy LES Moulineaux (FR)**

(74) Representative: **Plagnard, Thomas Eric**
**Withings SA**
**2, rue Maurice Hartmann**
**92130 Issy-Les-Moulineaux (FR)**

(56) References cited:
WO-A1-2017/214582    WO-A1-2018/112401
WO-A1-2018/171656    CN-A- 107 040 707
CN-A- 110 432 868    CN-U- 210 204 720
US-A1- 2017 281 027    US-A1- 2020 000 441

• **Anonymous: "Apple Watch - Wikipedia", , 31
March 2020 (2020-03-31), XP055717217,
Retrieved from the Internet:
URL:https://web.archive.org/web/2020033119
3156/https://en.wikipedia.org/wiki/Apple_W atch
[retrieved on 2020-07-22]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

This disclosure pertains to the field of devices allowing to measure a blood oxygenation level of an individual. More precisely, this disclosure is directed to an oximeter wristwatch, for measuring a blood oxygenation level at a wrist of an individual.

### Background Art

A technique for monitoring certain physiological parameters of a patient is commonly referred to as "pulse oximetry", and the devices built based upon pulse oximetry techniques are commonly referred to as pulse oximeters. Pulse oximetry may be used to measure various blood characteristics, such as the arterial blood oxygen saturation of hemoglobin (SaO2/SpO2). In practice SpO2 is usually referred as "Peripheral Oxygen Saturation", used as a proxy to replace an invasive measurement of arterial blood oxygen saturation (SaO2).

Often a technique termed photoplethysmography or, in short, "PPG" is used for obtaining the above physiological parameters. This involves a non-invasive optical sensor that detects the light response from within a patient's tissue indicative of the amount of light absorbed within the tissue at the illuminated site. One or more of the above physiological parameters such as blood oxygenation level may then be calculated based upon the amount of the absorbed light.

Pulse oximetry techniques typically utilize a tissue site that is well perfused with blood, such as a patient's finger, to place the sensor thereto. But having a clip like sensor at a finger strongly impedes the user from having a normal activity. And in addition, such sensor location requires the patient (or user) to be confined to a certain area, in close vicinity of an associated monitoring system, thereby limiting patient mobility.

There remains a need to provide an oximeter-enabled device that can be really worn as a non-invasive, user-friendly device.

The inventors have focused on a wrist worn device. Some devices use PPG technique to determine a user heart rate taken at the wrist. However, no watch-like device have so far achieved successfully to implement a pulse oximetry technique.

In US2014/0200423, it has already been proposed a wrist worn device to implement a pulse oximetry technique; however the disclosed device is bulky, expensive, not very comfortable to wear and not very convenient to wear. CN 110 432 868 A discloses a wearable device for measuring blood oxygen using green, red and infrared light.

The present inventors have worked in a direction of implementing reliable oximeter technique in a wristwatch.

### Summary of the disclosure

The invention is defined by an oximeter wristwatch according to claim 1. Further embodiments are disclosed by dependent claims **2-15**. Thanks to these dispositions, it is provided a long light path (reflective mode) within the wrist tissue, especially for the red and infrared measurements, where the path is substantially 10 mm long. Hence the reliability of the measurement is less influenced by various factors.

It should be noted that the promoted use of 3 different wavelengths and the minimal prescribed distances can overcome adverse condition like presence of tendon, hair, or other undesired element in the measurement path.

Regarding the term "photodiode" herein, it should be construed any type of light sensor sensitive to light rays, visible or beyond.

Regarding the term "selective photodiode", it should be understood here a photodiode sensitive at least to green wavelengths in the visible light; whereas infra-red wavelengths have to be discarded; hence "selective photodiode" could be likewise called "visible light photodiode".

The following features, can be optionally implemented, separately or in combination one with the others.

According to one aspect, the green LED can have a center emission wavelength comprised between 480 nm and 540nm, the red LED can have a center emission wavelength comprised between at 650 nm and 665 nm, and the infrared LED, can have a center emission wavelength comprised between at 920 nm and 960 nm. Thereby, using three spaced wavelengths brings a wide and rich data set, allowing to determine reliably a current blood oxygenation level (SpO2).

According to one aspect, the broadband photodiode and the selective photodiode have a rectangle shape. Thus, we can have a wide light collection area at the photodiode, arranged at the same space distance from the corresponding light source.

According to one aspect, the flat disk portion of the lens is delimited by a ring member forming a first ECG electrode, said ring member being part of the back-case assembly. Combination of PPG/SpO2 with ECG function is thus optimized.

According to one aspect, the lens only consists of said flat disk portion, made of glass, and glued onto the ring member. Thereby the lens is cheap to manufacture.

According to one aspect, the back-case assembly further comprises an annular cup member, wherein the annular cup member is generally axisymmetric around a watch axis, wherein the annular cup member forms a second ECG electrode. Mechatronics integration of two electrodes of the ECG function is thus optimized.

According to one aspect, the wristwatch may further comprise a flexible printed circuit (i.e. FPC), onto which are soldered the emission assembly, the broadband photodiode and the selective photodiode. Such a FPC is a convenient and thin solution for mechatronics integration. Further, such a FPC can connect elements that are not located at the same level.

According to one aspect, the ring member and the annular cup member are electrically connected to the flexible printed circuit for the ECG function. The FPC circuit handles all electrical connections involved in the back-case of the wristwatch.

According to one aspect, there is provided a silk mask etched/deposited against / next to an interior wall of the lens. This reduces optical crosstalk.

According to one aspect, there is provided partition walls in a PCB holder. This reduces optical crosstalk within the wristwatch.

According to one aspect, the flat disk portion of the lens protrudes (e1) from the back-case of the wristwatch. This provides good and homogenous optical contact with skin.

According to the invention, the emission assembly, the broadband photodiode and the selective photodiode are disposed according to an in-line arrangement, i.e. the selective photodiode is interposed between the emission assembly and the broadband photodiode. A first part of one light path is common with a part of a second light path. Further, the selective photodiode forms a barrier for direct path between LED and broadband photodiode.

According to one aspect, the emission assembly, the broadband photodiode and the selective photodiode have all a thickness less than 0.7 mm. The optical components are thin and occupy a small space. Having a single thickness, they can bear equally and evenly on the inner wall of the lens.

According to one aspect, each LED of the light emission assembly is part of a single SMD package LED assembly. This provides a compact and space-saving solution.

According to one aspect, each LED of the light emission assembly is selectively activated one after another. Wavelengths are timely segregated.

R15 According to one aspect, the control unit is configured to adaptively control the level of current flowing in each LED when each LED is selectively activated. The PPG function can be readily adapted to the skin nature and color of a large scope of users.

## Brief Description of Drawings

Other features, details and advantages will be shown in the following detailed description and on the figures, on which:

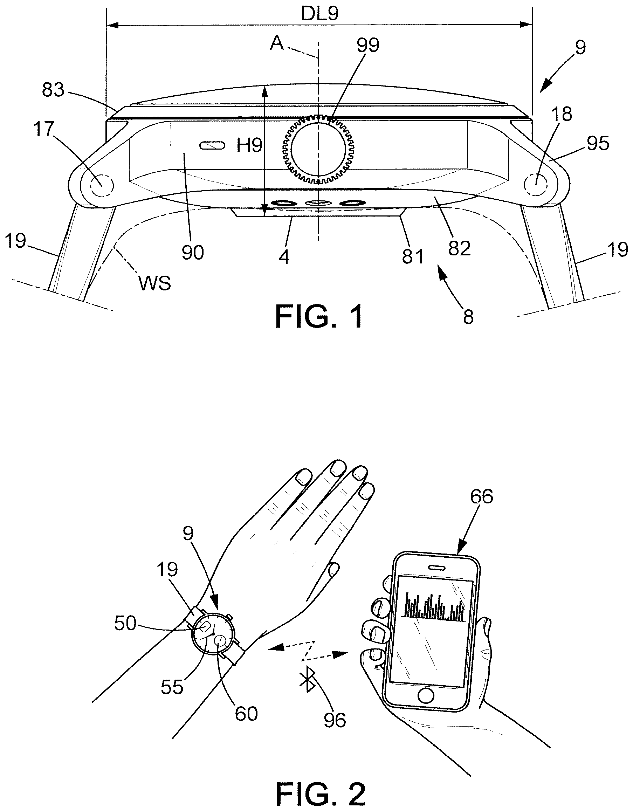

[Figure 1] is a side elevation view of an oximeter wristwatch according to the present disclosure.

[Figure 2] illustrates such a watch worn by a user at the wrist and having a communication interface.

[Figure 3] shows more in details a back view of the watch centered on the glass window.

[Figure 4] shows more in details an elevation sectional of the wristwatch back case bearing on the wrist skin.

[Figure 5] shows a detailed elevation sectional view of the glass window area.

[Figure 6] shows a bottom perspective view of the oximeter wristwatch of figure 1.

[Figure 7] shows a view of a wristwatch back case assembly.

[Figure 8] shows a partially cutaway view of the optical components at the glass window area.

[Figure 9] shows a functional block diagram of the oximeter wristwatch of figure 1.

[Figure 10] shows a time chart illustrating the selective activation of the various wavelengths LEDs.

**Description of Embodiments**

Overall

As shown on figures, an oximeter wristwatch **9** is provided. As will be exposed below, the wristwatch exhibits an oximeter function, but also an ECG function (i.e. Electro Cardio Gram).

We note here that the size of the watch **9** is small, it can fit all individuals, with large or small wrists. Main casing **90** has an overall diameter **DL9** less than 42 mm. Height of the watch denoted **H9** is generally comprised between 12 mm and 16 mm, in one embodiment it is comprised between 13 mm and 14 mm.

Main casing **90** exhibits two pairs of arms **93**, **95.** Each arm extends along a direction generally parallel to a longitudinal direction denoted **X.** At each pair of arm a shaft extends between the two free ends of the arms. Each of the shafts **17,18** extends along a direction generally parallel to a transverse direction denoted **Y.**

Main casing can be made of steel or high performance plastics. The front dial has a general watch axis denoted **A.**

A wristband **19** is removably attached to the pairs of arms **93**, **95.** The abovementioned shaft allows a pivot connection of the wrist band with respect to the main casing **90.**

As known for a wristwatch, there is provided current time display at the top/front face of the watch.

Here, there are provided physical hands **55**, each formed as a stick pivotally mounted around axis A. However hands different from physical stick(s) can also be considered in the present scope. A completely digital solution can also be envisioned.

There is provided a control knob **99.** This knob can be rotated around its axis and pushed along its axis.

There is provided a front bezel ring **83**, distinct from the main casing **90**, the purpose of which will be seen later.

Visible from the front side of the watch, there are provided two pixelated digitally controlled area **50**, **60.**

On said digitally controlled areas **50**, **60**, one displayed function is a pedometer function, other displayed data relate to or more bio-parameter(s). Some displayed functions are permanent, other are temporarily displayed.

Currently measured and/or lastly measured SpO2 (arterial blood oxygen saturation of hemoglobin) can be displayed in one of the digitally controlled area **50**, **60.** A result of an ECG measurement can also be temporarily displayed.

Back case assembly and optics

Attached to the main casing **90**, there is provided a back-case assembly generally denoted **8.** The back-case assembly **8** comprises, from the center, going radially outwards: a lens **4**, a ring member **81** and an annular cup member **82.**

The lens **4** forms a transparent or translucent window allowing optical interaction between the wristwatch and the environment at its back; notably the wrist skin **WS.**

The lens **4** comprises a flat disk portion, for good homogenous contact of the watch with the wrist skin surface.

According to one particular illustrated example, the lens **4** only consists of said flat disk portion. The lens **4** is made of glass. Its flat disk shape allows a very cost effective manufacturing. The lens flat disk portion has a diameter **DL4** comprised between 15 mm and 16,5 mm. The lens flat disk portion has a thickness comprised between 0,5 mm and 0,8 mm. In one embodiment, the lens flat disk portion has a thickness of 0,6 mm.

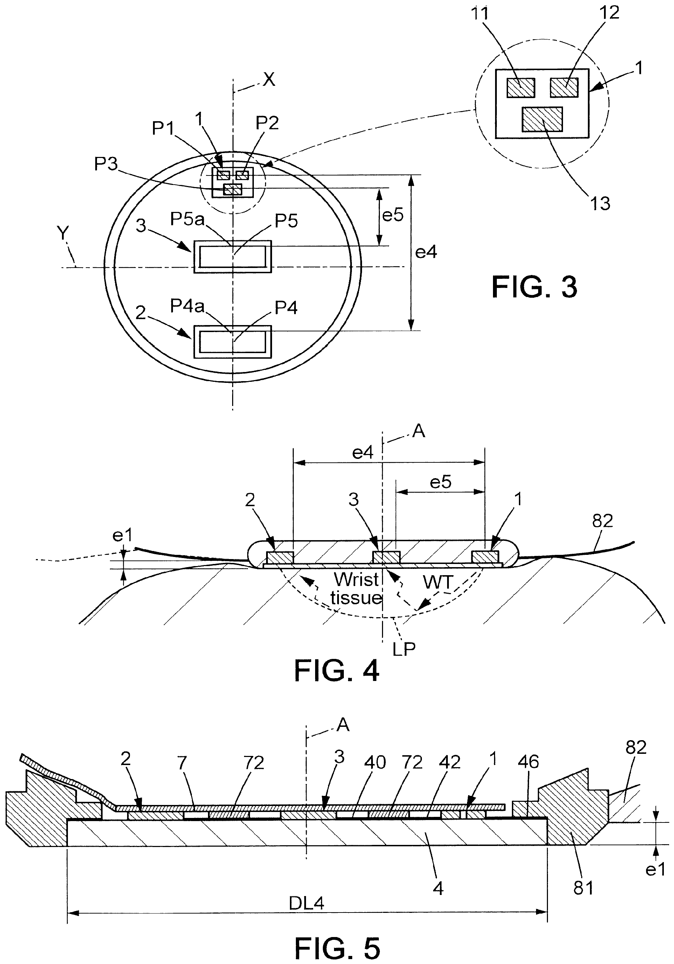

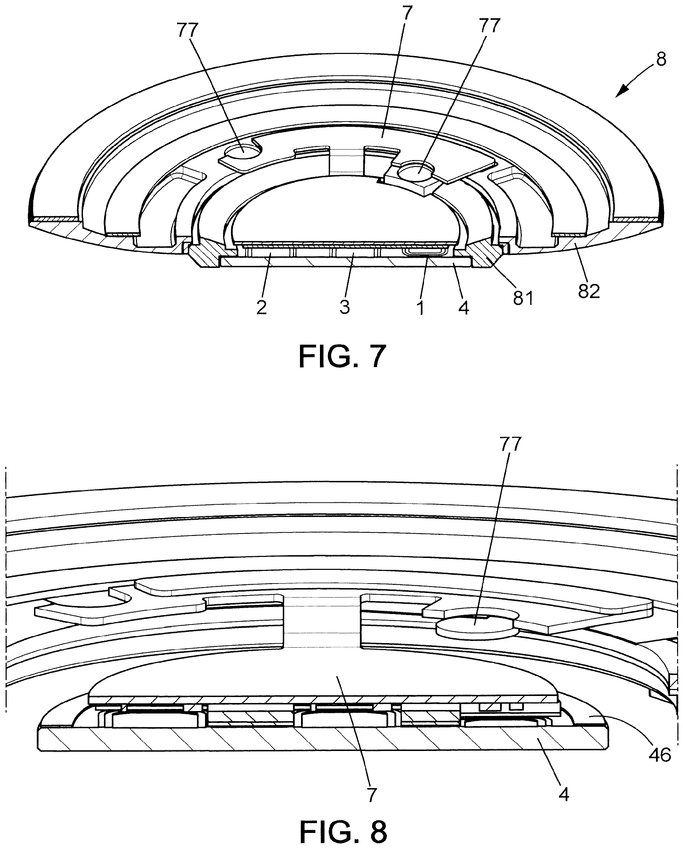

The lens 4 is glued onto the ring member **81.** An annular glue strip tape **46** is provided (cf Fig 5, Fig 8).

Behind the lens **4**, there is provided a light emission assembly **1.** The light emission assembly 1 comprises a green LED **13**, a red LED **12** and an infrared LED **11.** LED stands for Light Emission Diode.

In the present disclosure, the green LED **13** has a center emission wavelength comprised between 480 nm and 540 nm. According to one particular example, the green LED 13 has a center emission wavelength at **530** nm.

It is to be understood in the present disclosure that "green" has been given a broad scope, also encompassing partly of the blue wavelengths, at least the blue/green nuances.

In the present disclosure, the red LED **12** has a center emission wavelength comprised between **650** nm and **665** nm. According to one particular example, the red LED 12 has a center emission wavelength at **655** nm.

In the present disclosure, the infrared LED **11** has a center emission wavelength comprised between **920** nm and **960** nm. According to one particular example, the infrared LED 11 has a center emission wavelength at **940** nm.

Each LED is configured to emit light rays toward wrist tissue **WT** through the lens 4.

We note here that the above mentioned diode are conventional compact LEDs; they are not laser (i.e. they are not coherent light source) and therefore they are reasonably cheap components.

Further, there is provided a broadband photodiode **2** configured to receive red and infrared light rays. We already note that the Light Path denoted LP from the infrared and red LED (11,12) to the broadband photodiode 2 is somewhat 10 mm or more.

Further, there is provided a selective photodiode **3** configured to receive green (or blue) light rays. The selective photodiode is called selective since it eliminates infrared wavelengths. The selective photodiode can also eliminate red wavelengths. In a particular embodiment, the selective photodiode has a wavelength cutoff around 650 nm.

Advantageously, there are particular geometrical conditions that prevail in the proposed arrangement.

A distance denoted **e4** is taken from the emission point of infrared and red LED **11**, **12** to the first encountered border broadband photodiode **2**.

More precisely, the emission point of infrared LED 11 is at a first location denoted **P1**, i.e. the infrared LED **11** is arranged to emit light rays at the first position **P1**.

The emission point of red LED **12** is at a second location denoted **P2**, i.e. the red LED **12** is arranged to emit light rays at the second position denoted **P2**.

The emission point of green LED **13** is at a third location denoted **P3**, i.e. the green LED **13** is arranged to emit light rays at the third position denoted **P3**.

The broadband photodiode **2** is generally at a fourth location having a center position denoted **P4**. The selective photodiode **3** is generally at a fourth location having a center position denoted **P5**.

More precisely, the first encountered border of the selective photodiode **3**, from the green LED **13** and along longitudinal axis X is at location denoted **P5a**. Similarly the first encountered border of the broadband photodiode **2**, from the red LED 12 (respectively the infrared LED 11) and taken along longitudinal axis X is at location denoted **P4a**.

We note **e4** = Dist (P1,P4a) = Dist (P2,P4a)

It should be noticed that the light path for red light rays is nearly identical to the light path for infrared light rays. Indeed P1 and P2 are spaced by a distance less that a mm, and red / infrared light paths go to the same target namely the broadband photodiode **2**.

We note **e5** = Dist (P3,P5a)

In the illustrated example (cf Fig 4 and 5), **e4 is** at least 7 mm. In another example, **e4** > 7,5 mm. In another example, **e4** > 8 mm.

In the illustrated example, **e5** is at least 2 mm. In another example, **e5** > 2,4 mm. In another example, **e5** > 2.7 mm.

The broadband photodiode 2 and the selective photodiode 3 have a rectangle shape and a similar geometry. Long sides parallel to **Y**, short sides parallel to **X**. Overall, long sides have a length comprised between 3 mm and 3,4 mm, short sides have a length comprised between 1,8 mm and 2,2 mm. For light sensitive area, long sides have a length comprised between 2,4 mm and 3 mm, short sides have a length comprised between 1,2 mm and 1,6 mm.

The emission assembly 1, the broadband photodiode 2 and the selective photodiode 3 have all a same thickness. The thickness is preferably less than 0.7 mm.

Interestingly, the emission assembly 1, the broadband photodiode 2 and the selective photodiode 3 are disposed according to an in-line arrangement. Here, the selective photodiode 3 is interposed between the emission assembly 1 and the broadband photodiode 2 along the longitudinal axis X. However, another alignment direction is also considered. Since the lens is circular, all directions are equally possible.

We note that, thanks to the promoted in-line arrangement, the 'red' perfusion path within the wrist tissue encompasses the 'green' perfusion path within the wrist tissue. In other words, the first part of the light path are common for 'green' path and 'red/IR' path.

Each LED (11,12,13) of the light emission assembly 1 is part of a single SMD package LED assembly 1. Thus the three light sources occupy a very small space in the lens window.

We note here that the red and infrared LEDs are at a same position along X, side by side such that the light path to the broadband photodiode 2 is somehow identical. Also we note that the green LED is closer to the photodiode than the red and infrared LEDs.

On the inner wall **41** of the lens **4**, there is provided an opaque silk mask **42**. The silk mask 42 is placed against the interior wall of the lens. The silk mask 42 reduces optical crosstalk since no light can enter the inner space of the watch from outside at positions different from the broadband photodiode 2 and the selective photodiode 3.

The silk mask 42 can be deposited against the inner wall 41 of the lens and then etched to remove the mask at prescribed locations where the light rays are emitted on purpose and received on purpose for the PPG function.

In the back-case area, the wristwatch further comprise a flexible printed circuit **7** ('FPC' in short).The flexible printed circuit **7** comprises a plurality of electrical tracks, onto which are soldered the light emission assembly 1, the broadband photodiode 2 and the selective photodiode 3.

The flexible printed circuit **7** is also involved in the ECG function, the ring member **81** and the annular cup member **82** are electrically connected to the flexible printed circuit **7**. One connection point between the ring member **81** and the flexible printed circuit **7** is shown at ref **77** on figures 7 and 8.

Further, there are provided partition walls **72** interposed between optical components. More precisely, As shown at figure 5, there is arranged a partition wall **72** to optically isolate the light emission assembly 1 from the broadband photodiode **2** and the selective photodiode **3**. There is arranged a second partition wall **72** to optically isolate the broadband photodiode 2 from the light emission assembly and the selective photodiode 3. Such partition walls **72** complement functionally the above mentioned silk mask, to avoid any undesired optical leakage from the light source. Thereby the broadband photodiode 2 and the selective photodiode 3, when the wristwatch is worn at wrist, only receive perfused light through the wrist tissue WT.

Such partition walls **72** can be part of a FPC holder. This FPC holder can be a plastic part designed to be associated with the FPC to give a structure at some location of the FPC. The FPC holder can be fixed to the lens 4.

Further, the flat disk portion of the lens protrudes from the back-case of the wristwatch, by a protrusion distance denoted **e1.**The protrusion distance **e1** is comprised between 0,8 mm and 0,9 mm. It pushes gently on the wrist skin to ensure a good and homogenous optical contact with skin. A protrusion distance **e1** comprised between 0,9 mm and 1,2 mm is also considered.

The flat disk portion of the lens **4** is delimited by a ring member **81.** The ring member **81** forms a first ECG electrode.

Inner diameter of ring member **81** can be about 16 mm.

outer diameter of ring member **81** can be about 18 mm.

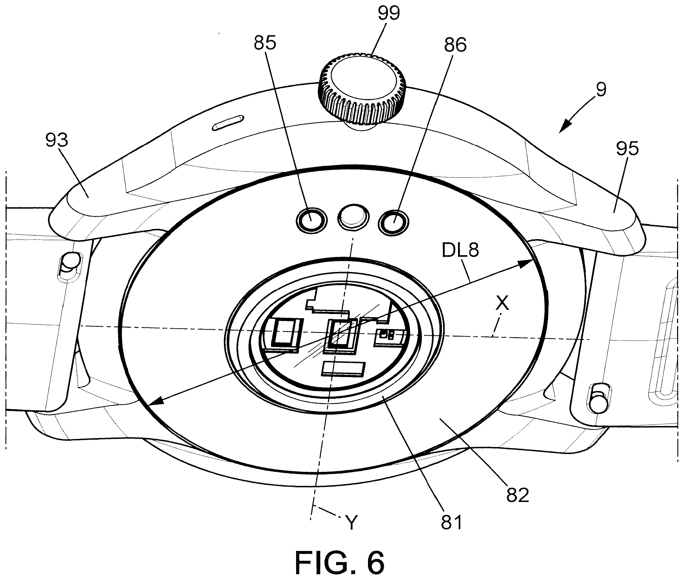

The back-case assembly **8** further comprises an annular cup member **82.** In one shown example, the annular cup member is generally axisymmetric around **A.** In one shown example, the annular cup member forms a second ECG electrode. The annular cup member **82**, exhibits an outer diameter comprised between 30 mm and 36 mm (cf Fig 6).

Further, there is provided a control unit **6.** The control unit **6** is housed in an upper area within the wristwatch. The control unit **6** is configured to handle the time display.

Further, the control unit **6** is configured to collect electrical signals delivered by the selective and broadband photodiodes (**2**, **3**) upon selective activation of each LED of the light emission assembly **1.** The control unit **6** is configured to calculate therefrom a current blood oxygenation level (SpO2).

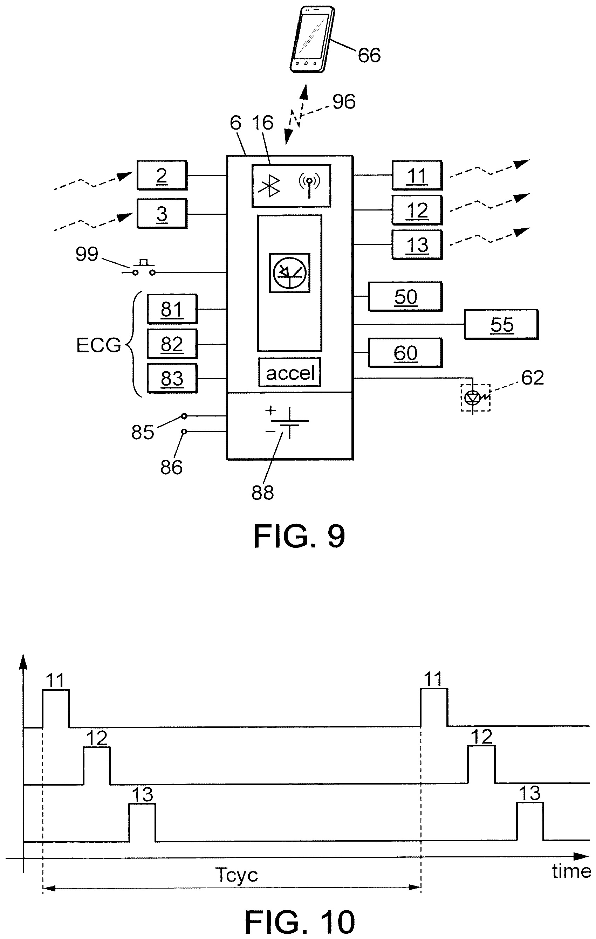

As shown at figure 9, the wristwatch comprises a battery **88.** The battery 88 is preferably a rechargeable type battery, for which there are provided charging pins **85, 86.**

Further, the wristwatch comprises an accelerometer device **15** and a wireless coupler **16.** Further, the wristwatch may comprise one or more night reading illumination light source **62.**

Functionality

As shown at figure 10, each LED of the light emission assembly 1 is selectively activated one after another. First the infrared LED 11, then the red LED 12, then green LED 13. Of course the order can be different. In one example, the full cycle lasts a duration denoted **Tcyc.** Tcyc can be 40 ms.

The emission power can be an adaptive parameter, depending on the skin color and/or thickness of the skin. There can be a calibration step, in which an average target at the receiving side is set and the emission power is varied such that the target at the receiving side is met. According to the skin nature and color, this calibration step can render the PPG subsequent measurements less sensitive and dependent on the user's skin.

Ambient light is also collected, when no LED is activated. If too much ambient light is received, it denotes that the wristwatch is not worn at all, or not worn correctly. Another way to detect a correct "worn" condition is using the LEDs and the reflected light therefrom.

The raw PPG signals are sampled at a sampling frequency, i.e. 25 Hz.

From the raw signal, there is provided, for each signal (infrared, red, green) a low pass filtering to extract a low pass component continuous or unmodulated component (DC1, DC2, DC3)

From that, a difference from raw signal to low pass component is performed to retrieve high pass component (AC1, AC2, AC3).

For each signal (infrared, red, green) a ratio is then computed, namely AC1/DC1 for infrared, AC2/DC2 for red and AC3/DC3 for green.

Therefrom, a blood oxygenation level is inferred with a F2 function, i.e. :

$$SpO2 = \mathbf{F2}\ (AC1/DC1, AC2/DC2, AC3/DC3)$$

Further, the wristwatch can be part of a system involving a remote entity like a smartphone **66**, with a short range communication link **96.** Results of the SpO2 measurement can be transmitted to the smartphone **66** and/or to a data server.

Further, the wristwatch has three ECG electrodes, two already discussed (81,82) at the back of the wristwatch. The third electrode **83**, named front bezel ring, is at the front face and can be sized by two fingers of the user's hand not wearing the wristwatch. A heart disorder can be found by an ECG analysis.

**Claims**

1. An oximeter wristwatch (9), for measuring a blood oxygenation level (SpO2) at a wrist of an individual (U), comprising :

    - a back-case assembly (8) with a lens (4), said lens comprising a flat disk portion,
    - a light emission assembly (1) comprising a green LED (13), a red LED (12) and an infrared LED (11), each configured to emit light rays toward wrist tissue (WT) through the lens,
    - a broadband photodiode (2) configured to receive red and infrared light rays,
    - a selective photodiode (3) configured to receive green light rays and to eliminate infrared wavelengths,
    - a control unit (6) configured to collect electrical signals delivered by the selective and broadband photodiodes (2,3) upon selective activation of each LED of the light emission assembly (1), and configured to calculate therefrom a current blood oxygenation level (SpO2),
    wherein the infrared LED (11) is arranged to emit light rays at a first position (P1), the red LED (12) is arranged to emit light rays at a second position (P2), the green LED (13) is arranged to emit light rays at a third position (P3),
    wherein the broadband photodiode (2) is arranged to receive light rays from a red and infrared path (LP) at a fourth position (P4,P4a), such that the broadband photodiode (2)
    is spaced from the first position of at least **e4**=7mm, and such that the broadband photodiode (2) is spaced from the second position of at least **e4**=7mm
    wherein the selective photodiode (3) is arranged to receive light rays from a green path at a fifth position (P5,P5a), such that the selective photodiode (3) is spaced from the third position of at least **e5**=2mm,
    wherein the emission assembly (1), the broadband photodiode (2) and the selective photodiode (3) are disposed according to an in-line arrangement, i.e. the selective photodiode (3) is interposed between the emission assembly (1) and the broadband photodiode (2),
    wherein the in-line arrangement is such that a first part of a light path in the wrist is common for the green path and the red and infrared path (LP),
    wherein the selective photodiode (3) forms a barrier for direct path between the light emission assembly (1) and broadband photodiode (2).

2. The oximeter wristwatch according to claim 1, wherein the green LED (13) has a center emission wavelength comprised between **480** nm and **540nm**, the red LED (12) has a center emission wavelength comprised between at **650** nm and **665** nm, and the infrared LED (11), has a center emission wavelength comprised between at **920** nm and **960** nm.

3. The oximeter wristwatch according to claim 1 or 2, wherein the broadband photodiode (2) and the selective photodiode (3) have a rectangle shape.

4. The oximeter wristwatch according to any of claims 1 to 3, wherein the flat disk portion of the lens is delimited by a ring member (81) forming a first ECG electrode, said ring member being part of the back-case assembly (8).

5. The oximeter wristwatch according to claim 4, wherein the lens only consists of said flat disk portion, made of glass, and glued onto the ring member (81).

6. The oximeter wristwatch according to claim 4 or 5, wherein the back-case assembly (8) further comprises an annular cup member (82), wherein the annular cup member is generally axisymmetric around a watch axis (A), wherein the annular cup member forms a second ECG electrode.

7. The oximeter wristwatch according to any of claims 1 to 6, wherein there is provided a silk mask deposited against an interior wall of the lens and etched to remove the mask at prescribed locations corresponding to the light emission assembly (1), to the broadband photodiode (2) and to the selective photodiode (3).

8. The oximeter wristwatch according to any of claims 1 to 7, wherein there is provided partition walls (72) in a PCB holder.

9. The oximeter wristwatch according to any of claims 1 to 8, wherein the flat disk portion of the lens protrudes (e1) from the back-case of the wristwatch.

10. The oximeter wristwatch of claim 9, wherein the lens consists of said flat disk portion, the lens being made of glass, the lens flat disk portion having a thickness comprised between 0.5 mm and 0.8 mm.

11. The oximeter wristwatch according to any one of claims 1 to 10, wherein the emission assembly (1), the broadband photodiode (2) and the selective photodiode (3) have all a thickness less than 0.7 mm.

12. The oximeter wristwatch according to any one of claims 1 to 11, wherein each **LED** of the light emission assembly (1) is selectively activated one after another.

13. The oximeter wristwatch according to any one of the preceding claims, in which the selective photodiode (3) is configured to eliminate red light rays.

14. The oximeter wristwatch according to any one of the preceding claims, wherein the selective photodiode has a wavelength cutoff around 650 nm.

15. The oximeter wristwatch according to any of claims 1 to 14, wherein **e4** > 7.5mm, or **e4** > 8 mm.


**Patentansprüche**

1. Oximeter-Armbanduhr (9) zum Messen eines Blutsauerstoffgehalts (SpO2) am Handgelenk einer Person (U), umfassend:

   - eine Gehäuserückseite (8) mit einer Linse (4), wobei die Linse einen flachen Scheibenabschnitt aufweist,
   - eine Lichtemissionsanordnung (1) mit einer grünen LED (13), einer roten LED (12) und einer Infrarot-LED (11), die jeweils so konfiguriert sind, dass sie durch die Linse Lichtstrahlen in Richtung des Handgelenksgewebes (WT) emittieren,
   - eine Breitband-Photodiode (2), die so konfiguriert ist, dass sie rote und infrarote Lichtstrahlen empfängt,
   - eine selektive Fotodiode (3), die so konfiguriert ist, dass sie grüne Lichtstrahlen empfängt und infrarote Wellenlängen eliminiert,
   - eine Steuereinheit (6), die so konfiguriert ist, dass sie elektrische Signale sammelt, die von den selektiven und breitbandigen Photodioden (2, 3) bei selektiver Aktivierung jeder LED der Lichtemissionsanordnung (1) geliefert werden, und so konfiguriert ist,
   dass sie daraus einen aktuellen Blutsauerstoffgehalt (SpO2) berechnet,
   wobei die Infrarot-LED (11) so angeordnet ist, dass sie Lichtstrahlen an einer ersten Position (P1) emittiert, die rote LED (12) so angeordnet ist, dass sie Lichtstrahlen an einer zweiten Position (P2) emittiert, die grüne LED (13) so angeordnet ist, dass sie Lichtstrahlen an einer dritten Position (P3) emittiert,
   wobei die Breitbandphotodiode (2) so angeordnet ist, dass sie Lichtstrahlen von einem Rot- und Infrarotpfad (LP) an einer vierten Position (P4, P4a) empfängt, so dass die Breitbandphotodiode (2) von der ersten Position um mindestens e4=7mm beabstandet ist, und so dass die Breitbandphotodiode (2) von der zweiten Position um mindestens e4=7mm beabstandet ist,
   wobei die selektive Fotodiode (3) so angeordnet ist, dass sie Lichtstrahlen von einem grünen Pfad an einer fünften Position (P5, P5a) empfängt, so dass die selektive Fotodiode (3) von der dritten Position um mindestens e5=2mm beabstandet ist,
   wobei die Emissionsbaugruppe (1), die Breitband-Photodiode (2) und die selektive Photodiode (3) gemäß einer In-Line-Anordnung angeordnet sind, d.h. die selektive Photodiode (3) ist zwischen der Emissionsbaugruppe (1) und der Breitband-Photodiode (2) angeordnet,
   wobei die In-Line-Anordnung so ist, dass ein erster Teil eines Lichtwegs im Handgelenk für den grünen Weg und den roten und infraroten Weg (LP) gemeinsam ist,
   wobei die selektive Fotodiode (3) eine Barriere für den direkten Weg zwischen der Lichtemissionsanordnung (1) und der Breitbandfotodiode (2) bildet.

2. Die Oximeter-Armbanduhr nach Anspruch 1, wobei die grüne LED (13) eine zentrale Emissionswellenlänge zwischen 480 nm und 540 nm hat, die rote LED (12) eine zentrale Emissionswellenlänge zwischen 650 nm und 665 nm hat und die Infrarot-LED (11) eine zentrale Emissionswellenlänge zwischen 920 nm und 960 nm hat.

3. Die Oximeter-Armbanduhr nach Anspruch 1 oder 2, wobei die Breitband-Photodiode (2) und die selektive Photodiode (3) eine rechteckige Form haben.

4. Die Oximeter-Armbanduhr nach einem der Ansprüche 1 bis 3, wobei der flache Scheibenabschnitt der Linse durch ein Ringelement (81) begrenzt ist, das eine erste EKG-Elektrode bildet, wobei das Ringelement Teil der hinteren Gehäuseanordnung (8) ist.)

**5.** Die Oximeter-Armbanduhr nach Anspruch 4, wobei die Linse nur aus dem flachen Scheibenteil besteht, der aus Glas besteht und auf das Ringelement (81) geklebt ist.)

**6.** Die Oximeter-Armbanduhr nach Anspruch 4 oder 5, wobei die hintere Gehäusebaugruppe (8) ferner ein ringförmiges Schalenelement (82) umfasst, wobei das ringförmige Schalenelement im Allgemeinen achsensymmetrisch um eine Uhrenachse (A) ist, wobei das ringförmige Schalenelement eine zweite EKG-Elektrode bildet.

**7.** Die Oximeter-Armbanduhr nach einem der Ansprüche 1 bis 6, wobei eine Seidenmaske vorgesehen ist, die gegen eine Innenwand der Linse aufgebracht und geätzt ist, um die Maske an vorgeschriebenen Stellen zu entfernen, die der Lichtemissionsanordnung (1), der Breitband-Photodiode (2) und der selektiven Photodiode (3) entsprechen.

**8.** Die Oximeter-Armbanduhr nach einem der Ansprüche 1 bis 7, wobei Trennwände (72) in einem Leiterplattenhalter vorgesehen sind.

**9.** Die Oximeter-Armbanduhr nach einem der Ansprüche 1 bis 8, wobei der flache Scheibenabschnitt der Linse aus dem hinteren Gehäuse der Armbanduhr herausragt (e1).

**10.** Die Oximeter-Armbanduhr nach Anspruch 9, wobei die Linse aus dem flachen Scheibenteil besteht, die Linse aus Glas hergestellt ist und der flache Scheibenteil der Linse eine Dicke zwischen 0,5 mm und 0,8 mm aufweist.

**11.** Die Oximeter-Armbanduhr nach einem der Ansprüche 1 bis 10, wobei die Emissionsanordnung (1), die Breitband-Photodiode (2) und die selektive Photodiode (3) alle eine Dicke von weniger als 0,7 mm aufweisen.

**12.** Die Oximeter-Armbanduhr nach einem der Ansprüche 1 bis 11, wobei jede LED der Lichtemissionsanordnung (1) selektiv nacheinander aktiviert wird.

**13.** Die Oximeter-Armbanduhr nach einem der vorhergehenden Ansprüche, bei der die selektive Photodiode (3) so konfiguriert ist, dass sie rote Lichtstrahlen eliminiert.

**14.** Die Oximeter-Armbanduhr nach einem der vorhergehenden Ansprüche, wobei die selektive Photodiode einen Wellenlängen-Cutoff um 650 nm aufweist.

**15.** Die Oximeter-Armbanduhr nach einem der Ansprüche 1 bis 14, wobei e4 > 7,5 mm oder e4 > 8 mm ist.

**Revendications**

**1.** Montre-bracelet oxymètre (9), pour mesurer un niveau d'oxygénation du sang (SpO2) au poignet d'un individu (U), comprenant :

- un ensemble boîtier arrière (8) avec une lentille (4), ladite lentille comprenant une portion de disque plat,
- un ensemble d'émission de lumière (1) comprenant une LED verte (13), une LED rouge (12) et une LED infrarouge (11), chacune configurée pour émettre des rayons lumineux vers le tissu du poignet (WT) à travers la lentille,
- une photodiode à large bande (2) configurée pour recevoir des rayons lumineux rouges et infrarouges,
- une photodiode sélective (3) configurée pour recevoir des rayons lumineux verts et pour éliminer les longueurs d'onde infrarouges,
- une unité de contrôle (6) configurée pour collecter les signaux électriques délivrés par les photodiodes sélectives et à large bande (2,3) lors de l'activation sélective de chaque LED de l'ensemble d'émission de lumière (1), et configurée pour calculer à partir de ceux-ci un niveau actuel d'oxygénation du sang (SpO2),
dans lequel la DEL infrarouge (11) est conçue pour émettre des rayons lumineux dans une première position (P1), la DEL rouge (12) est conçue pour émettre des rayons lumineux dans une deuxième position (P2), la DEL verte (13) est conçue pour émettre des rayons lumineux dans une troisième position (P3),
dans laquelle la photodiode à large bande (2) est conçue pour recevoir des rayons lumineux provenant d'un trajet rouge et infrarouge (LP) à une quatrième position (P4,P4a), de sorte que la photodiode à large bande (2) est espacée de la première position d'au moins e4=7mm, et de sorte que la photodiode à large bande (2) est espacée de la deuxième position d'au moins e4=7mm,
dans lequel la photodiode sélective (3) est conçue pour recevoir des rayons lumineux provenant d'un chemin vert à une cinquième position (P5,P5a), de sorte que la photodiode sélective (3) est espacée de la troisième position d'au moins e5=2mm,
l'ensemble d'émission (1), la photodiode à large bande (2) et la photodiode sélective (3) sont disposés en ligne, c'est-à-dire que la photodiode sélective (3) est interposée entre l'ensemble d'émission (1) et la photodiode à large bande (2),
dans laquelle la disposition en ligne est telle qu'une première partie d'un trajet lumineux dans le poignet est commune au trajet vert et aux trajets rouge et infrarouge (LP),
dans lequel la photodiode sélective (3) forme une barrière pour le trajet direct entre l'ensemble d'émission de lumière (1) et la photodiode à large bande (2).

2. Montre-bracelet oxymètre selon la revendication 1, dans laquelle la LED verte (13) a une longueur d'onde d'émission centrale comprise entre 480 nm et 540 nm, la LED rouge (12) a une longueur d'onde d'émission centrale comprise entre 650 nm et 665 nm, et la LED infrarouge (11) a une longueur d'onde d'émission centrale comprise entre 920 nm et 960 nm.

3. Montre-bracelet oxymètre selon la revendication 1 ou 2, dans laquelle la photodiode à large bande (2) et la photodiode sélective (3) ont une forme rectangulaire.

4. Montre-bracelet oxymètre selon l'une des revendications 1 à 3, dans laquelle la portion de disque plat de la lentille est délimitée par un anneau (81) formant une première électrode ECG, ledit anneau faisant partie de l'assemblage du boîtier arrière (8).

5. Montre-bracelet oxymètre selon la revendication 4, dans laquelle la lentille est uniquement constituée de la partie plate du disque, en verre, et collée sur l'anneau (81).

6. Montre-bracelet oxymètre selon la revendication 4 ou 5, dans laquelle l'assemblage du boîtier arrière (8) comprend en outre un élément annulaire (82), dans lequel l'élément annulaire est généralement axisymétrique autour d'un axe de montre (A), dans lequel l'élément annulaire forme une seconde électrode ECG.

7. Montre-bracelet oxymètre selon l'une des revendications 1 à 6, dans laquelle un masque de soie est déposé contre une paroi intérieure de la lentille et gravé pour enlever le masque aux endroits prescrits correspondant à l'ensemble d'émission de lumière (1), à la photodiode à large bande (2) et à la photodiode sélective (3).

8. Montre-bracelet oxymètre selon l'une des revendications 1 à 7, dans laquelle des parois de séparation (72) sont prévues dans un support de carte de circuit imprimé.

9. Montre-bracelet oxymètre selon l'une des revendications 1 à 8, dans laquelle le disque plat de la lentille fait saillie (e1) par rapport au boîtier arrière de la montre-bracelet.

10. Montre-bracelet oxymètre selon la revendication 9, dans laquelle la lentille est constituée de cette partie de disque plat, la lentille étant fabriquée en verre, la partie de disque plat de la lentille ayant une épaisseur comprise entre 0,5 mm et 0,8 mm.

11. Montre-bracelet oxymètre selon l'une des revendications 1 à 10, dans laquelle l'assemblage d'émission (1), la photodiode à large bande (2) et la photodiode sélective (3) ont tous une épaisseur inférieure à 0,7 mm.

12. Montre-bracelet oxymètre selon l'une des revendications 1 à 11, dans laquelle chaque DEL de l'ensemble d'émission de lumière (1) est activée sélectivement l'une après l'autre.

13. Montre-bracelet oxymètre selon l'une quelconque des revendications précédentes, dans laquelle la photodiode sélective (3) est configurée pour éliminer les rayons de lumière rouge.

14. Montre-bracelet oxymètre selon l'une quelconque des revendications précédentes, dans laquelle la photodiode sélective a une coupure de longueur d'onde autour de 650 nm.

15. Montre-bracelet oxymètre selon l'une des revendications 1 à 14, dans laquelle e4 > 7,5 mm, ou e4 > 8 mm.

**FIG. 1**

**FIG. 2**

FIG. 3

FIG. 4

FIG. 5

FIG. 6

FIG. 7

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140200423 A

- CN 110432868 A